# EUROPEAN PATENT SPECIFICATION

(11) **EP 0 852 619 B1**
(45) Date of publication and mention of the grant of the patent: **20.11.2002**
(21) Application number: 96932131.4
(22) Date of filing: 27.09.1996
(51) Int. Cl.: C12N 9/88, C12N 15/60

(54) **A MODIFIED GLUTAMIC ACID DECARBOXYLASE (GAD)**
MODIFIZIERTE GLUTAMINSÄURE DECARBOXYLASE (GAD)
DECARBOXYLASE D'ACIDE GLUTAMIQUE (DAG) MODIFIEE

(30) Priority: 29.09.1995 SE 9503379
(43) Date of publication of application: 15.07.1998
(73) Proprietor: Diamyd Therapeutics AB, 115 25 Stockholm (SE)
(72) Inventor: FALORNI, Alberto, I-06124 Perugia (IT); LERNMARK, Ake, S-216 11 Malmö (SE); ROBERTSON, John, S-131 38 Nacka (SE); ESSEN-MÖLLER, Anders, S-115 25 Stockholm (SE)
(74) Representative: Larsson, Karin
(86) International application number: SE9601210
(87) International publication number: WO97012034

(56) References cited:
- WO-A-92/05446
- WO-A-92/20811
- WO-A-95/27051

## Description

The present invention relates to a genetically-modified recombinant form of the 65 kDa human glutamic acid decarboxylase (hGAD65) that retains structural integrity for immuno-reactivity characteristic of the unmodified form, but that is without (or with considerably reduced) enzyme activity. The novel modified GAD is advantageously used in pharmaceutical compositions for treating autoimmune diseases.

### Background of the invention

The inhibitory neurotransmitter γ-aminobutyric acid (GABA), derived from L-glutamic acid by GAD is present in brain as well as several tissues outside the central nervous system. Biological functions of GAD and GABA extend beyond regulation of neurotransmission to include effects on the immune system as well as modulation of cell proliferation, protein synthesis and metabolism

GAD has recently been associated with autoimmune insulin-dependent diabetes mellitus (IDDM) (Boekkeskov et al, Nature 347:151 (1990), because of the increased incidence of IDDM in patients with stiff-man syndrome, a disease associated with autoantibodies against GAD. Antibodies from diabetic sera have also been shown to bind GAD. At least two different GAD, i.e. GAD 65 and 67 have been identified and sequenced in human beings. hGAD65 and hGAD67 have several epitopes in common. However, different epitopes are involved in different reactions and contexts.

Binding of the coenzyme pyridoxal-5'-phospate (PLP) to GAD was first reported by Roberts & Frankel in 1951 (J. Biol. Chem. 188:789), and formation of a Schiff base during GAD-mediated decarboxylation was subsequently proposed by W T Jenkins 1961, Fed. Proc. 20:978 (i.e. referenced in Roberts & Simonsen 1963, Biochem. Pharmacol. 12:113-134). Accordingly, PLP has been identified as a critical co-factor in decarboxylation by GAD. Cloning of GAD is disclosed in WO92/05446 and WO92/20811.

Location of the PLP binding site within the amino acid sequence of hGAD65 was enabled by publication of the cDNA sequence of this enzyme (P.N.A.S. 88:8337, fully incorporated in the description by reference). The site was found because of the homology centred around the amino acid lysine at a/a #396 in hGAD65 - to the sequence: X-His-Lys-X that was previously published as the PLP binding site in crystallised E.coli GAD (Biochemistry 9:226-233; Biochemistry 13:670-676).

This region in hGAD65 has the following amino acid sequence:

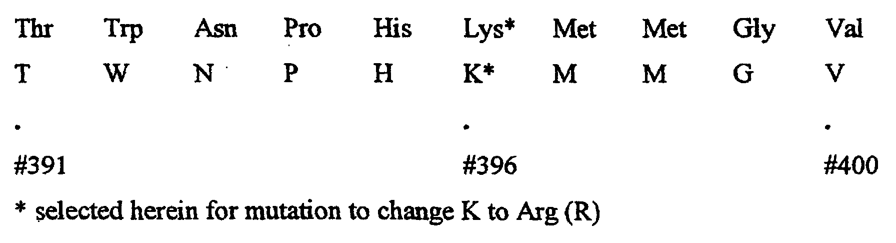

This homology in this region has subsequently been confirmed by Lernmark (unpublished) using computer homology matches to ca 10 other PLP-binding (non-GAD) proteins.

The approach of the present invention is a novel GAD without enzyme activity by mutating a single codon in the PLP binding site in the cDNA sequence for hGAD65. This results in the substitution of a single amino acid that is incapable of supporting wild-type enzyme activity.

As the lysine at #396 has been identified as critical for enzyme activity (via formation of a Schiff base during GAD-mediated decarboxylation), the present invention is directed to substitute an amino acid incapable of Schiff base formation at position #396 as a subtle means of achieving this effect.

In addition, a "conservative amino acid substitution" (i.e. replacement of lysine by an amino acid of comparable size and hydrophobicity) is intended to minimise any structural or conformational changes to the hGAD65 protein, and thereby avoid alterations to either antigenic etitopes or T cell determinants originally present in recombinant human GAD (rhGAD65).

### Summary of the invention

The present invention relates to a modified, non-enzymically active hGAD65 retaining the immunoreactive characteristics of wild type hGAD65, wherein amino acid #396 has been altered to an L-amino acid chosen from the group of isoleucine, arginine, glutamine, histidine or glycine. In a particularly preferred embodiment of the present invention amino acid #396 is altered to arginine.

### Detailed description of the invention

As already mentioned, the modified hGAD65 protein having considerably reduced enzymatic activity constitutes an object of the present invention. This altered protein can be used for treating auto-immune diseases, such as IDDM.

"Modified hGAD65 protein" as utilized herein refers to human glutamic acid decarboxylase having a molecular weight of 65 kDa wherein amino acid #396 has been altered to an amino acid chosen from the group of isoleucine, arginine, glutamine, histidine and glycine. Of these, arginine is considered to be most preferred as it is unable to form a Schiff base, but has similar hydrophobicity (index of both is 3.0) and size compared to lysine. The amino acid sequence of this modified hGAD65 protein is disclosed as SEQ. ID. NO. 3

The present invention also relates to nucleic acid sequences encoding a modified hGAD65 protein taken into account the degenerated genetic code. A preferred embodiment is disclosed as SEQ. ID. NO. 2.

In an other aspect, the present invention relates to an oligonucleotide that can be used for altering amino acid #396 to an amino acid chosen from the group of isoleucine, arginine, glutamine, histidine and glycine by site-directed mutagenesis of a nucleic acid encoding wild-type hGAD65. A preferred embodiment of such an oligonucleotide is disclosed as SEQ.ID.NO. 1.

In another aspect, the present invention relates to vectors and host cells comprising the above mentioned nucleic acids encoding modified hGAD65 protein. The nucleic acids may be readily introduced into a wide variety of host cells. Representative examples of such host cells include plant cells, eukaryotic cells and prokaryotic cells. Within preferred embodiments, the nucleic acid molecules are introduced into cells from an insect or a vertebrate or warm-blooded animal, such as a human, macaque, dog, cow, horse, pig, sheep, rat hamster, mouse, or a fish, or any hybrid thereof.

The nucleic acid molecules (or vectors) may be introduced into host cells by a wide variety of mechanisms, including for example calcium phosphate-mediated transfection (Wigler et al., Cell 14:725, 1978), lipofection; gene gun (Corsaro and Pearson, Somatic Cell Gen. 1:603, 1981; Graham and Van der Eb, Virology 52:456, 1973), electroporation (Neumann et al., EMBO J. 1:841-845, 1982), retroviral, adenoviral protoplast-mediated transfection or DEAE-dextran mediated transfection (Ausubel et al., (eds), Current Protocols in Molecular Biology, John Wiley and Sons, Inc., NY, NY, USA 1987) as well as baculovirus transfection, such as the method described in EP 0 327 180.

As noted above, the present invention also provides a variety of pharmaceutical compositions for treating and/or preventing autoimmune diseases, such as IDDM, comprising pharmaceutically active amount of a modified hGAD65 protein along with a pharmaceutically or physiologically acceptible carrier, excipients or diluents. Generally, such carriers should be non-toxic to recipients at the dosages and concentrations employed. Ordinarily, the preparation of such compositions entails combining the therapeutic agent with buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with non-specific serum albumin are exemplary appropriate diluents.

In addition, the pharmaceutical compositions of the present invention may be prepared for administration by a variety of routes, including for example orally, sublingually, rectally, intraarticularly, intracranially, intradermally, intramuscularly, intraocularly, intraperitoneally, intravenously or subcutaneously. The compositions can occur as tablets, sublingual tablets, granules, pills, capsules, dragées, solutions, syrups, mixtures, emulsions, suppositorys, aerosols, etc.

Furthermore, the above mentioned nucleic acid molecules can be used as pharmaceuticals and directly be injected into a human or animal in need of such treatment by using a gene gun (Corsaro and Pearson, Somatic Cell Gen. 1:603, 1981; Graham and Van der Eb, Virology 52:456, 1973). The treated human or animal body are then able to synthesize the modified hGAD65 according to the present invention.

As already mentioned, GAD is expressed by CNS, but also by islet cells in pancrease even producing insulin. GAD 65 is regarded to be the antigen initiating the immune response leading to diabetes, type 1 (IDDM). A GAD similar antigen has also been identified on Epstein Barr transformed lymphocytes and in coxsackievirus-infected cells. The influence of GAD 65 and the relation between IDDM and the presence of GAD 65 are described in Kaufman et al., Nature 361:69,1993; and November 4th, 1993, pp 69-71.

For the participation of GAD as an enzyme during the splitting of Glutamatic Acid into GABA and carbon dioxide the presence of a cofactor PLP (pyridoxal-5'-phosphate) is required. The PLP binding site consists of aminoacid 395-397 of wild type hGAD65. By replacing the aminoacid #396 with another amino acid the binding site for PLP is influenced so that said cofactor PLP is not bound to said binding site on the GAD molecule resulting in that said GAD molecule no longer participates in the GABA synthesis. The modification at said binding site is not influencing the GAD epitope being diabetogenerating. The modified GAD molecule is expressed with a known recombinant technique.

The manufacturing of cDNA from mRNA is well known today. The modified hGAD65 protein produced is then used to treat IDDM.

This modified hGAD65 so obtained is useful as an immunomodulant therapeutic to prevent certain autoimmune diseases (including insulin-dependent diabetes mellitus) and certain other diseases (including neurological diseases). The provision of this is a novel means to avoid (or minimise) the risk of toxicity (e.g. neurotoxicity) possibly arising from the enzyme activity of a GAD-based immuno-therapeutic.

From now on, the invention will be described with reference to the enclosed examples. However, it should be understood that the scope of the present invention is not to be restricted to those examples.

### Materials and methods

### 1. DNA oligomer design and synthesis

Both oligos (below) represent sequences from the coding strand of both the insert or vector, and are intended to hybridise to the same (ie. non-coding) strand of p65 (Synectics proprietary clone derived from the cDNA sequence for human GAD65 published by Karlsen et al 1991 Proc. Natl. Acad. Sci USA 88:8337-8341 and Bu et al. 1992 Proc. Natl. Acad. Sci USA 89:2115-2119).

The mutagenic primer (SEQ. ID. NO.1) was designed according to Stratagene recommendations and contained the CGC codon for Arginine, flanked at either end by 14 nts derived from the corresponding hGAD65 cDNA sequence. Of the 6 alternative codons for Arginine (viz. AGA, AGG, CGA, CGC, CGG, CGU), the codon CGC was selected as this has the greatest usage in animals (Grantham R, Gautier C, Gouy M, Jacobzone M, Mercier R 1981 Nucleic Acid Res 9:43-74). Accordingly, the 31-mer mutagenic oligo has sequence: -corresponding to nt #1186 (upstream end) and #1216 (downstream) of p65. This mutagenic primer (coded "SDM-PLP") was synthesised at the Karonlinska Institute facility, and kinased (via T4 polynycleotide kinase, see Current Protocols in Molecular Biology, John Wiley & Sons, Inc.), prior to use. The Tm of SDM-PLP is estimated as 60°C and was used during mutagenesis.

The Sca I to Mlu I selection primer was chosen because of the absence of sites in the rhGAD65 insert and the single site in the pGEM4z vector of p65. This kinased primer was supplied by Stratagene (catalogue #300331).

### 2. Site-directed mutagenesis of proprietary hGAD65 clone "p65"

The Chameleon ™ double-stranded DNA site-directed mutagenesis kit from Stratagene was used for modification of the Synectics Biotechnology rhGAD65 clone (coded "p65"), and followed the manufacturers' instructions in the instruction manual (Catalog #200509, June 13, 1994).

### 3. DNA sequencing of candidate clones

The required mutation (present in clone #18) was confirmed by DNA sequencing using "Taq DyeDeoxy Terminator Cycle Sequencing" (Applied BioSystems) and "ALF" sequenator at the Karonlinska facility. The sequencing oligo "JR11" (annealing to nts #1353-1369 in the coding strand) was used to confirm sequence at nts "1200-1202. Oligo "JR4" (annealing to the noncoding strand upstream of nts "1200-1202, at nts #1033-1050) was used to obtain sequence in a downstream direction to the site of the mutation.

Methods used for these manipulations followed previously-published procedures (e.g. Current Protocols in Molecular Biology, John Wiley & Sons, Inc; Sambrook, Fritsch & Maniatis Molecular Cloning: A Laboratory Manual. Cold Spring Harbor Laboratory Press, 1989).

### 4. Check for comparable immunoreactivity between mutant and wild-type

Immunoreactivity of the *in vitro* transcribed/translated mutant rhGAD65 cDNA (clone #18) was shown to be indistinguishable from the original (non-mutant) p65 clone by using IDDM sera in a radioimmunoassay specific for GAD65 autoantibodies (Falorni et al. 1993 Diabetologia 36 (suppl. 1) A45; Falomi et al. 1994 Autoimmunity 19:113-125).

This analysis compared immunoreactivity of the mutant and wild-type clones by comparing radioactivity precipitated by ca. 20GAD65-positive IDDM sera on incubation with ³⁵S-labelled rhGAD65 protein synthesised *in vitro* from either the wild-type or mutant rhGAD65 cDNA clones.

### Example 1: Site-directed mutagenesis of wild-type hGAD65

An oligonucleotide having the sequence: was synthesized, phosphorylated and used to mutate a cDNA having the sequence of fig. 2 in Karlsen et al., P.N.A.S. 88:8337, 1991 according to the instructions in the above mentioned Chamelon™ kit. Competent Escherichia coli F'11 rec A cells were transformed with p65 mutant plasmids. Positive clones were screened by DNA sequencing using the Taq DyeDeoxy™ Terminator Cycle Sequencing Kit from Applied Biosystems. A clone containing the desired mutant was found.

### Example 2: In vitro translation of ³⁵S-mutant hGAD65

Recombinant modified human GAD65 was produced by in vitro transcription/translation of the cDNA inserted into the pGEM4z vector. The translation reaction was carried out using SP6 RNA polymerase and rabbit reticulocyte lysate. The materials used in the experiment were part of the TNT coupled transcription/translation system from Promega.

The following reaction mixture was prepared:

| | |
|---|---|
| Plasmid DNA encoding modified hGAD65 (2mg/ml) | 1 µl |
| RNAsin (Promega) | 1 µl |
| TNT buffer | 2 µl |
| Amino acid mixture (without methionine)(Promega) | 1 µl |
| ³⁵S-methionine (NEN-Dupont) | 4 µl |
| SP6 RNA polymerase (Promega) | 1 µl |
| Rabbit Reticulocyte lysate (Promega) | 25 µl |
| Water | up to 50 µl |

The reactions were incubated for 90 minutes at 30°C. At the end of the incubation time, 2 ml from the reaction tube was incubated for 10 minutes at 37°C with 1M NaOH/2% H₂O₂ and 30 minutes on ice with 0.9 ml 25% trichloroacetic acid (TCA) in order to precipitate proteins that may have been formed. The TCA precipitate was collected on a GF-A Whatman glass fibre filter and its radioactivity was evaluated in a liquid scintillation analyser. It was shown that the precipitate was radioactive and hence, modified hGAD65 was formed

### Example 3: Immunoprecipitation of 35S-modified hGAD65 with IDDM sera

In vitro translated modified hGAD65 from example 2 was immunoprecipitated with IDDM sera to test the immunoreactivity. For each serum sample 15000 cpm TCA-precipitable GAD65 was precipitated with 2 µl of human serum from healthy or diabetic individuals (final serum dilution 1:50). After overnight immunoprecipitation in cold room, antibody bound ³⁵S-modified hGAD65 was separated from free ³⁵S-modified hGAD65 by protein A Sepharose using a Multiscreen Assay Millipore system. Immunoprecipitated radioactivity was evaluated in a liquid scintillation analyzer.

Immunoprecipitation of ³⁵S-modified hGAD65 for diabetic sera was significantly higher than that obtained with sera from healthy persons. The used diabetic sera were previously found positive for antibodies against wild type hGAD65. Hence, it was concluded that modified hGAD65 has an immunoreactivity similar to that of wild type hGAD65.

## Claims

1. A modified glutamic acid decarboxylase having amino acid sequence: wherein Xxx represents a L amino acid chosen from the group of isoleucine, arginine, glutamine, histidine and glycine.

2. A modified glutamic acid decarboxylase according to claim 1, **characterized in that** Xxx is arginine.

3. A nucleotide sequence encoding a glutamic acid decarboxylase according to any of claims 1-2.

4. A nucleotide sequence according to claim 3 which is the one disclosed herein as SEQ. ID. NO. 2.

5. A pharmaceutical composition comprising a therapeutically effective amount of a modified glutamic acid decarboxylase according to any of claims 1-2 together with a pharmaceutically acceptable carrier, excipient and/or diluent.

6. Use of a modified glutamic acid decarboxylase according to any of claims 1-2 for preparing a pharmaceutical composition for treating and/or preventing autoimmune disorders, such as IDDM.

7. A nucleotide sequence according to any of claims 3-4 for medical use.

8. A nucleotide sequence according to any of claims 3-4 for genetic vaccination.

9. An oligonucleotide for modifying glutamic acid decarboxylase, **characterized in that** it encodes the aminoacid sequence wherein Xxx represents a L amino acid chosen from the group of isoleucine, arginine, glutamine, histidine and glycine.

10. An oligonucleotide according to claim 9 having the sequence disclosed as SEQ.ID.No.1.

11. A vector containing the nucleic acid sequence according to any of claims 3-4.

12. A host cell containing a vector according to claim 11.

13. A procedure for producing a modified glutamic acid decarboxylase according to any of claims 1-2 **characterized in** cultivating a host cell according to claim 12 under suitable conditions.

## Patentansprüche

1. Modifizierte Glutaminsäuredecarboxylase mit der Aminosäuresequenz: worin Xxx eine L-Aminosäure repräsentiert, welche aus der Gruppe ausgewählt ist, welche aus Isoleucin, Arginin, Glutamin, Histidin und Glycin besteht.

2. Modifizierte Glutaminsäuredecarboxylase nach Anspruch 1, **dadurch gekennzeichnet, dass** Xxx Arginin ist.

3. Nukleotidsequenz, welche eine Glutaminsäuredecarboxylase nach einem der Ansprüche 1 oder 2 kodiert.

4. Nukleotidsequenz nach Anspruch 3, welche hier offenbart ist als SEQ.ID.NO. 2.

5. Pharmazeutische Zusammensetzung, umfassend eine therapeutisch wirksame Menge einer modifizierten Glutaminsäuredecarboxylase nach einem der Ansprüche 1 - 2 zusammen mit einem pharmazeutisch geeigneten Trägerstoff, Arzneimittelträger und/oder Streckmittel.

6. Verwendung einer modifizierten Glutaminsäuredecarboxylase nach einem der Ansprüche 1 - 2 zur Herstellung einer pharmazeutischen Zusammensetzung zur Behandlung und/oder zum Verhindern von Autoimmunstörungen, wie z.B. IDDM.

7. Nukleotidsequenz nach einem der Ansprüche 3 - 4 für medizinische Zwekke.

8. Nukleotidsequenz nach einem der Ansprüche 3 - 4 für eine genetische Impfung.

9. Oligonukleotid zum Modifizieren von Glutaminsäuredecarboxylase, **gekennzeichnet dadurch, dass** es die Aminosäuresequenz kodiert, worin Xxx eine L-Aminosäure repräsentiert, welche aus der Gruppe von Isoleucin, Arginin, Glutamin, Histidin und Glycin ausgewählt ist.

10. Oligonukleotid nach Anspruch 9 mit einer Sequenz, die offenbart ist als SEQ.ID.No.1.

11. Vektor enthaltend eine Nukleinsäuresequenz nach einem der Ansprüche 3 - 4.

12. Wirtszelle, enthaltend einen Vektor nach Anspruch 11.

13. Verfahren zur Herstellung einer modifizierten Glutaminsäuredecarboxylase nach einem der Ansprüche 1 - 2, **gekennzeichnet durch** das Kultivieren einer Wirtszelle nach Anspruch 12 unter geeigneten Bedingungen.

## Revendications

1. Acide glutamique décarboxylase modifiée, dont la séquence d'acides aminés est la suivante : dans laquelle Xxx représente un résidu de L-aminoacide choisi parmi l'isoleucine, l'arginine, la glutamine, l'histidine et la glycine.

2. Acide glutamique décarboxylase modifiée, conforme à la revendication 1, **caractérisée en ce que** Xxx représente un résidu d'arginine.

3. Séquence de nucléotides codant une acide glutamique décarboxylase modifiée, conforme à l'une des revendications 1 et 2.

4. Séquence de nucléotides conforme à la revendication 3, qui est celle qui est donnée ici en tant que Séquence n° 2.

5. Composition pharmaceutique comprenant une certaine quantité, efficace du point de vue thérapeutique, d'une acide glutamique décarboxylase modifiée, conforme à l'une des revendications 1 et 2, ainsi qu'un véhicule, excipient et/ou diluant admissible en pharmacie.

6. Emploi d'une acide glutamique décarboxylase modifiée, conforme à l'une des revendications 1 et 2, dans la préparation d'une composition pharmaceutique destiné au traitement ou à la prévention d'une maladie auto-immune comme le DSID (diabète sucré insulino-dépendant).

7. Séquence de nucléotides conforme à l'une des revendications 3 et 4, destinée à un usage médical.

8. Séquence de nucléotides conforme à l'une des revendications 3 et 4, destinée à une vaccination génique

9. Oligonucléotide permettant de modifier l'acide glutamique décarboxylase, **caractérisé en ce qu'**il code la séquence d'acides aminés suivante : dans laquelle Xxx représente le résidu d'un L-aminoacide choisi parmi l'isoleucine, l'arginine, la glutamine, l'histidine et la glycine.

10. Oligonucléotide conforme à la revendication 9, qui présente la séquence donnée en tant que Séquence n° 1.

11. Vecteur comportant une séquence d'acide nucléique conforme à l'une des revendications 3 et 4.

12. Cellule hôte contenant un vecteur conforme à la revendication 11.

13. Procédé de production d'une acide glutamique décarboxylase modifiée conforme à l'une des revendications 1 et 2, **caractérisé en ce que** l'on cultive, dans des conditions appropriées, une cellule hôte conforme à la revendication 12.


### SEQUENCE LISTING

(1) GENERAL INFORMATION:
   (i) APPLICANT:
      (A) NAME: Synetics Medical AB
      (B) STREET: Renstiernas gata 12
      (C) CITY: Stockholm
      (E) COUNTRY: Sweden
      (F) POSTAL CODE (ZIP): 116 28
   (ii) TITLE OF INVENTION: A modified glutamic acid decarboxylase.
   (iii) NUMBER OF SEQUENCES: 3
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: SE 9503379-1
(2) INFORMATION FOR SEQ ID NO: 1:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO: 1:
   CGTGGAATCC ACACCGCATG ATGGGAGTCC C 31
(2) INFORMATION FOR SEQ ID NO: 2:
   (i) SEQUENCE CHARACTERISTICS:
      (A) LENGTH: 1761 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO: 2:
   ATGGCATCTC CGGGCTCTGG CTTTTGGTCT TTCGGGTCGG AAGATGGCTC TGGGGATTCC 60
   GAGAATCCCG GCACAGCGCG AGCCTGGTGC CAAGTGGCTC AGAAGTTCAC GGGCGGCATC 120
   GGAAACAAAC TGTGCGCCCT GCTCTACGGA GACGCCGAGA AGCCGGCGGA GAGCGGCGGG 180
   AGCCAACCCC CGCGGGCCGC CGCCCGGAAG GCCGCCTGCG CCTGCGACCA GAAGCCCTGC 240
   AGCTGCTCCA AAGTGGATGT CAACTACGCG TTTCTCCATG CAACAGACCT GCTGCCGGCG 300
   TGTGATGGAG AAAGGCCCAC TTTGGCGTTT CTGCAAGATG TTATGAACAT TTTACTTCAG 360
   TATGTGGTGA AAAGTTTCGA TAGATCAACC AAAGTGATTG ATTTCCATTA TCCTAATGAG 420
   CTTCTCCAAG AATATAATTG GGAATTGGCA GACCAACCAC AAAATTTGGA GGAAATTTTG 480
   ATGCATTGCC AAACAACTCT AAAATATGCA ATTAAAACAG GGCATCCTAG ATACTTCAAT 540
   CAACTTTCTA CTGGTTTGGA TATGGTTGGA TTAGCAGCAG ACTGGCTGAC ATCAACAGCA 600
   AATACTAACA TGTTCACCTA TGAAATTGCT CCAGTATTTG TGCTTTTGGA ATATGTCACA 660
   CTAAAGAAAA TGAGAGAAAT CATTGGCTGG CCAGGGGGCT CTGGCCATGG GATATTTTCT 720
   CCCGGTGGCG CCATATCTAA CATGTATGCC ATGATGATCG CAGCGTTTAA GATGTTCCCA 780
   GAAGTCAAGG AGAAAGGAAT GGCTGCTCTT CCCAGGCTCA TTGCCTTCAC GTCTGAACAT 840
   AGTCATTTTT CTCTCAAGAA GGGAGCTGCA GCCGTAGGGA TTGGAACAGA CAGCGTGATT 900
   CTGATTAAAT GTGATGAGAG AGGGAAAATG ATTCCATCTG ATCTTGAAAG AAGGATTCTT 960
   GAAGCCAAAC AGAAAGGGTT TGTTCCTTTC CTCGTGAGTG CCACAGCTGG AACCACCGTG 1020
   TACGGAGCAT TTGACCCCCT CTTAGCTGTC GCTGACATTT GCAAAAAGTA TAAGATCTGG 1080
   ATGCATGTGG ATGCAGCTTG GGGTGGGGGA TTACTGATGT CCCGAAAACA CAAGTGGAAA 1140
   CTGAGTGGCG TGGAGAGGGC CAACTCTGTG ACGTGGAATC CACACCGCAT GATGGGAGTC 1200
   CCTTTGCAGT GCTCTGCTCT CCTGGTTAGA GAAGAGGGAT TGATGCAGAA TTGCAACCAA 1260
   ATGCATGCCT CCTACCTCTT TCAGCAAGAT AAACATTATG ACCTGTCCTA TGACACTGGA 1320
   GACAAGGCCT TACAGTGCGG ACGCCACGTT GATGTTTTTA AACTATGGCT GATGTGGAGG 1380
   GCAAAGGGGA CTACCGGGTT TGAAGCGCAT GTTGATAAAT GTTTGGAGTT GGCAGAGTAT 1440
   TTATACAACA TCATAAAAAA CCGAGAAGGA TATGAGATGG TGTTTGATGG GAAGCCTCAG 1500
   CACACAAATG TCTGCTTCTG GTACATTCCT CCAAGCTTGC GTACTCTGGA AGACAATGAA 1560
   GAGAGAATGA GTCGCCTCTC GAAGGTGGCT CCAGTGATTA AAGCCAGAAT GATGGAGTAT 1620
   GGAACCACAA TGGTCAGCTA CCAACCCTTG GGAGACAAGG TCAATTTCTT CCGCATGGTC 1680
   ATCTCAAACC CAGCGGCAAC TCACCAAGAC ATTGACTTCC TGATTGAAGA AATAGAACGC 1740
   CTTGGACAAG ATTTATAATA A 1761
(2) INFORMATION FOR SEQ ID NO: 3:
   (i) SEQUENCE CHARACTERISTICS:
      (A) LENGTH: 585 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (xi) SEQUENCE DESCRIPTION: SEQ ID NO: 3:
   Met Ala Ser Pro Gly Ser Gly Phe Trp Ser Phe Gly Ser Glu Asp Gly
   1 5 10 15
   Ser Gly Asp Ser Glu Asn Pro Gly Thr Ala Arg Ala Trp Cys Gln Val
   20 25 30
   Ala Gln Lys Phe Thr Gly Gly Ile Gly Asn Lys Leu Cys Ala Leu Leu
   35 40 45
   Tyr Gly Asp Ala Glu Lys Pro Ala Glu Ser Gly Gly Ser Gln Pro Pro
   50 55 60
   Arg Ala Ala Ala Arg Lys Ala Ala Cys Ala Cys Asp Gln Lys Pro Cys
   65 70 75 80
   Ser Cys Ser Lys Val Asp Val Asn Tyr Ala Phe Leu His Ala Thr Asp
   85 90 95
   Leu Leu Pro Ala Cys Asp Gly Glu Arg Pro Thr Leu Ala Phe Leu Gln
   100 105 110
   Asp Val Met Asn Ile Leu Leu Gln Tyr Val Val Lys Ser Phe Asp Arg
   115 120 125
   Ser Thr Lys Val Ile Asp Phe His Tyr Pro Asn Glu Leu Leu Gln Glu
   130 135 140
   Tyr Asn Trp Glu Leu Ala Asp Gln Pro Gln Asn Leu Glu Glu Ile Leu
   145 150 155 160
   Met His Cys Gln Thr Thr Leu Lys Tyr Ala Ile Lys Thr Gly His Pro
   165 170 175
   Arg Tyr Phe Asn Gln Leu Ser Thr Gly Leu Asp Met Val Gly Leu Ala
   180 185 190
   Ala Asp Trp Leu Thr Ser Thr Ala Asn Thr Asn Met Phe Thr Tyr Glu
   195 200 205
   Ile Ala Pro Val Phe Val Leu Leu Glu Tyr Val Thr Leu Lys Lys Met
   210 215 220
   Arg Glu Ile Ile Gly Trp Pro Gly Gly Ser Gly Asp Gly Ile Phe Ser
   225 230 235 240
   Pro Gly Gly Ala Ile Ser Asn Met Tyr Ala Met Met Ile Ala Arg Phe
   245 250 255
   Lys Met Phe Pro Glu Val Lys Glu Lys Gly Met Ala Ala Leu Pro Arg
   260 265 270
   Leu Ile Ala Phe Thr Ser Glu His Ser His Phe Ser Leu Lys Lys Gly
   275 280 285
   Ala Ala Ala Leu Gly Ile Gly Thr Asp Ser Val Ile Leu Ile Lys Cys
   290 295 300
   Asp Glu Arg Gly Lys Met Ile Pro Ser Asp Leu Glu Arg Arg Ile Leu
   305 310 315 320
   Glu Ala Lys Gln Lys Gly Phe Val Pro Phe Leu Val Ser Ala Thr Ala
   325 330 335
   Gly Thr Thr Val Tyr Gly Ala Phe Asp Pro Leu Leu Ala Val Ala Asp
   340 345 350
   Ile Cys Lys Lys Tyr Lys Ile Trp Met His Val Asp Ala Ala Trp Gly
   355 360 365
   Gly Gly Leu Leu Met Ser Arg Lys His Lys Trp Lys Leu Ser Gly Val
   370 375 380
   Glu Arg Ala Asn Ser Val Thr Trp Asn Pro His Arg Met Met Gly Val
   385 390 395 400
   Pro Leu Gln Cys Ser Ala Leu Leu Val Arg Glu Glu Gly Leu Met Gln
   405 410 415
   Asn Cys Asn Gln Met His Ala Ser Tyr Leu Phe Gln Gln Asp Lys His
   420 425 430
   Tyr Asp Leu Ser Tyr Asp Thr Gly Asp Lys Ala Leu Gln Cys Gly Arg
   435 440 445
   His Val Asp Val Phe Lys Leu Trp Leu Met Trp Arg Ala Lys Gly Thr
   450 455 460
   Thr Gly Phe Glu Ala His Val Asp Lys Cys Leu Glu Leu Ala Glu Tyr
   465 470 475 480
   Leu Tyr Asn Ile Ile Lys Asn Arg Glu Gly Tyr Glu Met Val Phe Asp
   485 490 495
   Gly Lys Pro Gln His Thr Asn Val Cys Phe Trp Tyr Ile Pro Pro Ser
   500 505 510
   Leu Arg Thr Leu Glu Asp Asn Glu Glu Arg Met Ser Arg Leu Ser Lys
   515 520 525
   Val Ala Pro Val Ile Lys Ala Arg Met Met Glu Tyr Gly Thr Thr Met
   530 535 540
   Val Ser Tyr Gln Pro Leu Gly Asp Lys Val Asn Phe Phe Arg Met Val
   545 550 555 560
   Ile Ser Asn Pro Ala Ala Thr His Gln Asp Ile Asp Phe Leu Ile Glu
   565 570 575
   Glu Ile Glu Arg Leu Gly Gln Asp Leu
   580 585